# FASCICULE DE BREVET EUROPEEN

(11) **EP 3 175 234 B1**
(45) Date de publication et mention de la délivrance du brevet: **14.06.2023**
(21) Numéro de dépôt: 15762737.3
(22) Date de dépôt: 28.07.2015
(51) Int. Cl.: G01N 33/18

(54) **SYSTEME DE MESURE INTELLIGENT AU POINT DE LIVRAISON D'UN FLUIDE**
INTELLIGENTES MESSSYSTEM AM ORT DER ZUGABE EINER FLÜSSIGKEIT
INTELLIGENT MEASURING SYSTEM LOCATED AT POINT OF DELIVERY OF A FLUID

(30) Priorité: 30.07.2014 FR 1457363
(43) Date de publication de la demande: 07.06.2017
(73) Titulaire: SUEZ International, 92040 Paris La Défense Cedex (FR)
(72) Inventeur: CAMPAN, Francis, 92160 Antony (FR)
(74) Mandataire: IPAZ
(86) Numéro de dépôt international: PCT/IB2015/055689
(87) Numéro de publication internationale: WO 2016/016803

(56) Documents cités:
- EP-A1- 2 966 438
- US-A- 5 581 189
- US-A1- 2008 052 012
- US-A1- 2010 085 211
- US-A1- 2010 085 211
- US-A1- 2013 205 879

## Description

### Domaine technique

La présente invention se rapporte au domaine des dispositifs de mesure en ligne d'au moins un paramètre physique, chimique ou biologique d'un fluide, en particulier de l'eau ou du gaz, s'écoulant dans une conduite d'un système d'approvisionnement de ce fluide, en particulier dans un réseau de distribution.

La présente invention concerne plus particulièrement, mais non exclusivement, de tels dispositifs installés au point de livraison du fluide, le point de livraison désignant le point du réseau où le fluide est livré à un utilisateur.

### Etat de la technique antérieure

On connaît dans l'art antérieur différents équipements destinés à mesurer un ou plusieurs paramètres d'un fluide s'écoulant dans un réseau de distribution, en particulier dans le domaine de la distribution d'eau potable.

Un premier type d'équipements connus est une sonde munie d'un ensemble de capteurs aptes à détecter des paramètres tels que le chlore, la conductivité, la pression ou encore la température du fluide.

Le brevet US 8,479,598 B2 décrit une sonde comprenant un corps muni d'une tête de mesure, en particulier de forme cylindrique, ainsi que plusieurs capteurs montés à l'une des extrémités de cette tête de mesure. La tête de mesure comprend des logements agencés pour pouvoir y insérer des capteurs de manière axiale ou radiale.

Une telle sonde multi-capteurs est typiquement montée dans une conduite par insertion de la tête de mesure dans une direction radiale par rapport à la conduite.

L'insertion d'une sonde multi-capteurs dans une conduite implique de réaliser une ouverture sur la périphérie de la conduite, par exemple un trou cylindrique fileté permettant de recevoir un pas de vis formé sur le corps de la sonde.

Une sonde multi-capteurs comprend typiquement des moyens de collecte de données de mesures réalisées par les capteurs et des moyens de communication de ces données.

Par exemple, le produit Intellisonde^{™} de la société « Intellitect Water » permet de mesurer et d'enregistrer des valeurs mesurées par un ou plusieurs capteurs à intervalles réguliers, par exemple toutes les heures ou toutes les minutes. Les valeurs mesurées sont par exemple transmises par une norme informatique de type réseau sans fil (par exemple GPRS) ou par protocole ethernet. En termes d'autonomie, ce produit est équipé de piles électriques embarquées lui assurant un fonctionnement typiquement pendant une période de six mois.

La sonde multi-capteurs possède plusieurs inconvénients. Son coût complet de possession est élevé car, en plus du coût d'achat, son bon fonctionnement nécessite notamment des opérations de recalibration ou de changement de pièces. De plus, son autonomie énergétique est limitée au regard des fréquences d'enregistrement et de communication des données de mesures souhaitées et ses dimensions ne permettent pas de l'installer sur la plupart des conduites au point de livraison.

Ces inconvénients rendent la sonde multi-capteurs peu appropriée au déploiement de ce type d'équipement à des points de livraison de fluide.

Un second type d'équipements connus concerne un système pour la télé-relève de compteurs de fluide, notamment de compteurs d'eau, tel que décrit dans le brevet FR 2 929 752 B1. Ce document décrit un système de relevé et de transmission de valeurs mesurées par au moins un capteur de mesure. Ce système comprend un émetteur pour la transmission de valeurs de mesures et un récepteur, typiquement installé à distance, apte à recueillir les valeurs transmises par l'émetteur.

Un tel système de télé-relève est typiquement alimenté par une pile électrique.

Le moyen d'alimentation (pile) d'un système de télé-relève autorise une fréquence d'enregistrement et de communication de données de mesures relativement faible, typiquement de l'ordre de une transmission par jour, pour atteindre une autonomie énergétique satisfaisante, par exemple de l'ordre de plusieurs années. Un tel moyen d'alimentation est cependant insuffisant pour réaliser une collecte de mesures supplémentaires, par exemple à l'aide d'une sonde multi-paramètres.

En outre, les équipements qui viennent d'être décrits sont généralement équipés de programmes de traitement de signal très simples, rendant peu optimal le paramétrage des opérations de mesure et de traitement des données de mesure, et augmentant en conséquence les coûts globaux de fonctionnement.

Le document US-A-20080052012 décrit un dispositif de mesure de la conductivité électrique d'eau en déplacement dans un conduit d'écoulement appartenant à un réseau de distribution de l'eau. Un tel dispositif est destiné à être installé au domicile d'un usager, en particulier au niveau d'un conduit d'écoulement d'un évier se trouvant dans une cuisine, pour lui permettre de contrôler la qualité de l'eau livrée au domicile. Le dispositif est installé en coupure sur le conduit au point de livraison de l'eau. Ce dispositif comprend un capteur positionné avec son extrémité de mesure qui s'étend dans le conduit d'écoulement pour mesurer un paramètre physique chimique ou biologique de l'eau.

Le document EP2966438 fait partie de l'art antérieur selon l'Article 54(3) CBE et divulgue un dispositif de mesure et de signalisation calorimétriques des grandeurs physico- chimiques de l'eau pour la surveillance et l'entretien de l'eau des piscines. En particulier, une pluralité de capteurs électrochimiques sont immergés (figure 2) dans l'eau à analyser à partir d'un même élément de support modulaire faisant partie de la conduite de l'écoulement de l'eau. Cependant, le circuit électronique de commande ne correspond pas à un module intelligent apte à déclencher au moins une mesure par l'au moins un capteur à intervalles de temps déterminés, et diagnostiquer une anomalie d'au moins un paramètre physique, chimique ou biologique du fluide en fonction d'au moins une valeur mesurée.

Un but de la présente invention est de proposer un dispositif de mesure d'au moins un paramètre d'un fluide qui soit énergétiquement autonome, notamment afin de pouvoir l'installer en tout point de réseaux de distribution existants, y compris lorsqu'aucune source d'énergie externe n'est disponible.

Un autre but de la présente invention est de proposer un dispositif de mesure d'au moins un paramètre d'un fluide pouvant être installé à un point de livraison du fluide.

Encore un autre but de la présente invention est de proposer un dispositif de mesure d'au moins un paramètre d'un fluide capable de mesurer différents paramètres physiques, biologiques ou chimiques du fluide.

La présente invention a aussi pour but de proposer un dispositif de mesure d'au moins un paramètre d'un fluide apte à transmettre des informations à un terminal informatique distant, notamment afin de signaler des anomalies de distribution ou encore des modifications de propriétés du fluide.

Un autre but de la présente invention est de proposer un dispositif de mesure d'au moins un paramètre d'un fluide doté d'une intelligence embarquée, notamment afin de détecter des anomalies dans la distribution ou encore des modifications de propriétés du fluide, et de transmettre une alerte liée à une éventuelle anomalie dès lors que celle-ci se produit.

Encore un autre but de la présente invention est de proposer un dispositif de mesure d'au moins un paramètre d'un fluide pouvant être paramétré à distance.

La présente invention a aussi pour but de proposer un dispositif de mesure d'au moins un paramètre d'un fluide ayant une autonomie énergétique optimisée, en particulier pour permettre de réaliser des mesures et de transmettre des données de mesure à des fréquences importantes.

Cette invention a aussi pour but de proposer un dispositif de mesure d'au moins un paramètre d'un fluide capable de transmettre des données de mesure en temps réel.

Plus généralement, la présente invention a pour but de proposer un dispositif de mesure d'au moins un paramètre d'un fluide facilitant les opérations d'installation et de maintenance, et réduisant les contraintes d'exploitation.

### Exposé de l'invention

Cet objectif est atteint avec un Dispositif de mesure d'au moins un paramètre physique, chimique ou biologique d'un fluide selon la revendication 1.

La section interne moyenne du conduit d'écoulement est sensiblement identique à la section interne moyenne de la conduite.

Un avantage d'un tel dispositif est qu'il peut être fabriqué en un nombre de séries limitées dans lesquelles la section interne moyenne correspond aux sections internes moyennes typiques des conduites des réseaux de distribution du fluide existants, par exemple d'un diamètre de 20 mm.

La configuration « en ligne », c'est-à-dire par connexion du conduit d'écoulement dans le prolongement de la conduite, facilite grandement son installation (voir plus loin le procédé d'installation associé à ce dispositif).

Selon une autre particularité avantageuse de l'invention, le module intelligent comprend en outre des moyens de communication aptes à réaliser une communication informatique entre le module intelligent et un terminal informatique distant, le module intelligent étant agencé pour transmettre au terminal informatique une information relative à l'anomalie diagnostiquée.

Selon encore une autre particularité avantageuse de l'invention, l'instant de la transmission de l'information relative à l'anomalie diagnostiquée est déterminé par l'instant auquel ladite anomalie est diagnostiquée. Autrement dit, cette information est transmise dès qu'une anomalie est diagnostiquée, ou après un laps de temps très court.

Ou encore, le module intelligent est agencé pour transmettre au terminal informatique une information relative à l'anomalie diagnostiquée à intervalles de temps réguliers dans la condition où une anomalie est diagnostiquée, c'est-à-dire que l'information est transmise si et seulement si une anomalie est diagnostiquée.

La présence d'un module intelligent embarqué capable de traiter localement des données de mesure permet d'éviter de réaliser un tel traitement au sein d'un terminal informatique distant.

Ainsi, le dispositif selon l'invention permet notamment d'optimiser l'utilisation des ressources énergétiques en rendant possible une émission sélective de données de mesures ou d'une information d'alerte, par exemple lorsque le module intelligent diagnostique une anomalie.

De plus, un tel dispositif permet de transmettre une information d'anomalie dans un délai éventuellement très court, voire de manière quasi-instantanée, dès lors qu'une anomalie est diagnostiquée par le dispositif, plutôt qu'à intervalles de temps réguliers.

Avantageusement, les moyens de communication comprennent au moins un émetteur compatible avec des systèmes de réception disponibles dans le périmètre géographique du réseau de distribution, autorisant une communication bidirectionnelle entre le module intelligent et le terminal informatique distant.

En particulier, l'invention prévoit que le module intelligent peut être paramétré depuis le terminal informatique distant.

Un paramétrage à distance permet de réduire les coûts opérationnels en évitant à un opérateur souhaitant par exemple re-paramétrer ou mettre à jour un logiciel implémenté dans le module intelligent de se déplacer sur le lieu d'installation du dispositif.

Selon le dispositif comprend en outre un élément de support modulaire agencé pour permettre de positionner au moins un capteur de manière à ce que son extrémité de mesure soit reçue dans l'au moins un des logements d'insertion, cet élément de support modulaire comportant des moyens de fixation de l'au moins un capteur aptes à maintenir l'au moins un capteur dans une position de mesure dans laquelle l'au moins un capteur est apte à mesurer l'au moins un paramètre physique, chimique ou biologique du fluide s'écoulant à travers le conduit d'écoulement.

La modularité de l'élément de support permet de changer aisément un capteur, et de standardiser les moyens de fixation des capteurs.

Selon d'autre particularités avantageuses, l'au moins un capteur est un capteur de type débit, et, éventuellement, le capteur de débit est utilisé en tant que compteur de télé-relève.

L'inclusion de la fonction de télé-relève au sein d'un tel dispositif permet de faire l'économie d'une installation de compteur de télé-relève classique. Cela permet aussi de substituer la fonction de comptage réalisée par un compteur classique par une mesure d'un paramètre physique d'un fluide éventuellement plus sensible.

Selon une autre particularité avantageuse, la fréquence de mesure de l'au moins un capteur est paramétrable, et cette fréquence de mesure peut être paramétrée séparément pour chacun des capteurs lorsque le dispositif comprend plusieurs capteurs.

De cette façon, le rapport entre la dépense énergétique requise pour réaliser une mesure et la fréquence de mesure pertinente peut être optimisé.

Selon encore une autre particularité avantageuse, le dispositif comprend en outre un système de fourniture d'énergie additionnelle apte à récupérer de l'énergie présente dans l'environnement dans lequel est installé le dispositif, un système de conversion de cette énergie récupérée en énergie électrique et un système d'alimentation apte à alimenter le dispositif avec cette énergie électrique.

Cette particularité permet d'augmenter la durée d'autonomie du dispositif.

Ne faisant pas partie de la présente invention, un procédé d'installation d'un tel dispositif est aussi décrit et est caractérisé en ce qu'il comprend :
- une étape de découpe de la conduite de manière à y réaliser une ouverture et à former le premier tronçon et le deuxième tronçon,
- une étape de positionnement du dispositif par alignement de la première extrémité du conduit d'écoulement à une extrémité du premier tronçon et de la deuxième extrémité du conduit d'écoulement à une extrémité du deuxième tronçon,
- une étape de fixation du dispositif dans la position alignée décrite à l'étape précédente à l'aide des moyens de fixation.

### Description de la figure et modes de réalisation

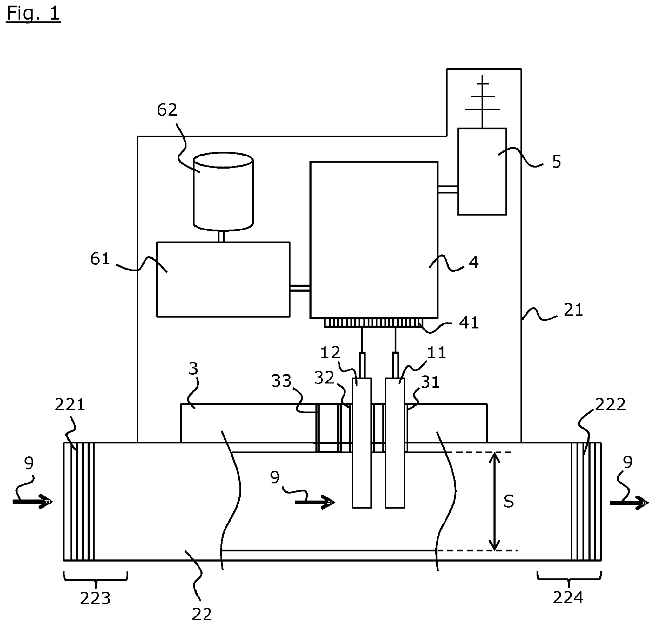

D'autres avantages et particularités de l'invention apparaîtront à la lecture de la description détaillée de mises en oeuvre et de modes de réalisation, et de la FIGURE 1 annexée qui représente un exemple de réalisation du dispositif selon l'invention.

La FIGURE 1 représente le dispositif selon un mode de réalisation actuellement préféré de l'invention.

Dans cet exemple, le dispositif comprend un corps 21 renfermant, notamment, des capteurs 11, 12, un système d'alimentation 61, 62 électrique, des moyens de communication 5 et un module intelligent 4.

### Conduit d'écoulement

Le corps 21 est constitué d'un conduit d'écoulement 22 au travers duquel s'écoule un fluide 9 (de la gauche vers la droite sur la FIGURE 1). Le conduit d'écoulement 22 est représenté en coupe partielle faisant apparaître l'intérieur du conduit d'écoulement 22 dans une région dans laquelle sont situés les capteurs 11, 12 (voir plus loin).

Le conduit d'écoulement 22 consiste ici en un élément tubulaire de diamètre intérieur S, par exemple réalisé en polychlorure de vinyle.

### Assemblage du dispositif avec une conduite

Le conduit d'écoulement 22 comprend deux extrémités opposées 223, 224 dépassant vers l'extérieur du corps 21. Des filetages 221, 222 sont formés sur la surface externe du conduit d'écoulement 22 au niveau de ses extrémités 223, 224. Ces filetages 221, 222 formant vis constituent des moyens de fixation permettant d'assembler le dispositif avec des tronçons de conduite respectifs, par exemple par l'intermédiaire d'éléments de raccord comportant une partie filetée intérieure formant écrou.

Ces moyens de fixation 221, 222 sont utilisés pour assembler le conduit d'écoulement 22 dans une position en ligne au sein d'une conduite. Par exemple, on coupe un tronçon de la conduite sur une longueur correspondant sensiblement à la longueur du conduit d'écoulement 22, on aligne le conduit d'écoulement 22 avec la conduite de sorte que le conduit d'écoulement 22 soit situé à la place du tronçon coupé, puis on fixe le dispositif par l'intermédiaire des moyens de fixation 221, 222 et de raccords adaptés.

Lorsque le dispositif est ainsi installé, le conduit d'écoulement 22 constitue une partie de la conduite de sorte que, lorsqu'un fluide 9 s'écoule dans la conduite d'un point A situé en amont du dispositif à un point B situé en aval du dispositif, ce fluide 9 s'écoule à travers le conduit d'écoulement 22.

### Capteurs

Le conduit d'écoulement 22 est représenté solidaire d'un élément de support modulaire 3 (FIGURE 1). L'élément de support modulaire 3 comprend des orifices alignés avec des orifices de dimension sensiblement identique qui sont réalisés dans le conduit d'écoulement 22. Ces orifices sont des logements d'insertion 31, 32, 33 agencés pour recevoir des capteurs 11, 12. Le logement d'insertion 33 est représenté sans capteur en FIGURE 1.

Les capteurs 11, 12 sont fixés à l'élément de support modulaire 3 par tous moyens de fixation adaptés qui dépendent par exemple de leur fabrication. Par exemple, les capteurs 11, 12 ont un corps partiellement fileté permettant de les positionner et les visser sur un écrou formé dans les logements d'insertion 31, 32, et on utilise des contre-écrous pour maintenir les capteurs 11, 12.

En référence à la FIGURE 1, les capteurs 11, 12 sont fixées dans une position dans laquelle leur extrémité de mesure, c'est-à-dire l'extrémité comprenant un moyen sensible à un paramètre du fluide 9, est située à l'intérieur du conduit d'écoulement 22.

Les capteurs 11, 12 sont de préférence montés de manière étanche dans les logements d'insertion 31, 32, par exemple par utilisation de joints toriques, afin d'éviter qu'une partie du fluide 9 pénètre dans l'espace du corps 1 dans lequel se trouve notamment le module intelligent 4.

L'élément de support modulaire 3 est agencé pour permettre de monter un ou plusieurs capteurs de manière modulaire. Des bouchons peuvent être prévus pour obstruer les logements d'insertion n'accueillant pas de capteur.

Les capteurs 11, 12 sont par exemple des capteurs de mesure d'une ou plusieurs propriétés physiques de l'eau (p. ex. volume, vitesse d'écoulement, débit, pression, niveau de bruit, etc.) ou encore des capteurs de mesure de la qualité de l'eau par quantification de paramètres chimiques ou biologiques (p. ex. température, chlore, conductivité, oxygène dissous, pH, potentiel Redox, matières organiques, micro-polluants, métaux, sous-produits de désinfection, etc.).

Les capteurs 11, 12 sont connectés au module intelligent 4 par branchement sur le gestionnaire d'entrée-sortie 41.

### Alimentation

Le système d'alimentation 61, 62 fournit de l'énergie électrique aux capteurs 11, 12, aux moyens de communication 5, au module intelligent 4 ainsi qu'à des modules électroniques, par exemple de conditionnement, de calcul et de stockage (non représentés).

Le système d'alimentation 61, 62 assure la fourniture d'une tension stabilisée en termes de courant requis par chacun de ces modules, moyens et capteurs.

Le système d'alimentation comprend de préférence une pile électrique 62 reliée à un module de gestion d'énergie 61 capable de transférer de l'énergie électrique au module intelligent 4.

Dans un mode de réalisation alternatif, le module de gestion d'énergie 61 est relié à une batterie d'accumulateurs 62 et à un système de fourniture d'énergie additionnelle. De manière non limitative, ce système est un récupérateur d'énergie, par exemple récupérant de l'énergie cinétique produite par l'écoulement du fluide 9 dans le conduit d'écoulement 22 à l'aide d'une turbine, ou utilisant de l'énergie récupérée avec un différentiel de température entre la conduite et le tampon d'un regard en chaussée.

### Communication

Les moyens de communication 5 sont agencés pour échanger des données entre le module intelligent et un terminal informatique distant ou point d'accès (récepteur radio privé ou réseau public).

Le terminal informatique distant est par exemple situé dans des locaux du fournisseur du fluide 9.

Les moyens de communication 5 sont d'un type capable de mettre en forme des données à émettre depuis le module intelligent jusqu'au terminal informatique ou point d'accès et de décoder des données reçues conformément à un protocole d'échange de trames avec le point d'accès.

Les moyens de communication 5 peuvent comprendre un module de sécurisation des échanges par des techniques du type cryptage ou authentification.

### Module intelligent

Le module intelligent 4, formant une unité de traitement, comprend de préférence un processeur et une mémoire capables de traiter des données de mesures réalisées par les capteurs 11, 12 et d'ordonnancer des tâches à réaliser par chacun des composants reliés au module intelligent 4.

De préférence, le module intelligent 4 comprend un système d'exploitation de type temps réel et des codes logiciels capables de réaliser des opérations de traitement avancées, par exemple :
- conditionnement de signaux mesurés par les capteurs 11, 12,
- stockage d'échantillons horodatés pour chaque paramètre mesuré (profondeur configurable),
- détection d'événement sur chaque série temporelle des mesures de capteurs,
- traitement spécifique tel que :
   o déclenchement d'un envoi d'une série temporelle lorsqu'un événement ou une anomalie est détectée,
   o application par exemple de fonctions statistiques ou de méthodes spectrales sur un signal pour une consolidation dans des modèles utilisés par l'exploitant du dispositif.

De préférence, le module intelligent embarque aussi un composant apte à paramétrer ou configurer le dispositif.

Par exemple, des paramétrages peuvent être réalisés par un terminal informatique connecté au dispositif localement via une connexion physique, et des paramétrages peuvent être réalisés par un terminal informatique distant communiquant avec le module intelligent à l'aide de moyens de communication 5 tels que décrits plus haut.

Des exemples d'actions à distance sont :
- chargement d'un code de traitement spécifique,
- fréquence d'échantillonnage des capteurs 11, 12,
- fréquence de transmission de données de mesure vers un terminal informatique distant,
- type de traitement ou action lorsqu'un événement ou une anomalie est détectée,
- mise à jour de logiciel embarqué,
- etc.

## Revendications

1. Dispositif de mesure d'au moins un paramètre physique, chimique ou biologique d'un fluide (9), en particulier de l'eau ou du gaz, s'écoulant dans une conduite appartenant à un réseau de distribution du fluide, ce dispositif étant destiné à être installé en coupure sur ladite conduite au point de livraison du fluide, ladite conduite comportant ainsi un premier tronçon et un deuxième tronçon, ce dispositif comprenant :
- au moins un capteur (11, 12) muni d'une extrémité de mesure,
- un corps (21) comportant une ouverture formant un conduit d'écoulement (22), et au moins un logement d'insertion (31, 32, 33) apte à recevoir ladite extrémité de mesure de l'au moins un capteur (11, 12), le logement d'insertion (31, 32, 33) débouchant dans le conduit d'écoulement (22),
- des moyens de fixation (221, 222) agencés pour connecter :
o une première extrémité (223) du conduit d'écoulement (22) à une extrémité du premier tronçon,
o une deuxième extrémité (224) du conduit d'écoulement (22) à une extrémité du deuxième tronçon,
de manière à permettre au fluide (9) de s'écouler à travers le conduit d'écoulement (22), et
un module intelligent (4) étant apte à déclencher au moins une mesure par l'au moins un capteur à intervalles de temps déterminés, et diagnostiquer une anomalie d'au moins un paramètre physique, chimique ou biologique du fluide en fonction d'au moins une valeur mesurée,
**caractérisé en ce qu'**il comprend en outre un élément de support modulaire (3) comprenant des orifices alignés avec des orifices de dimensions sensiblement identiques réalisés dans le conduit d'écoulement (22), ces orifices qui sont des logements d'insertion (31, 32, 33) étant agencés pour permettre de positionner l'au moins un capteur (11, 12) de manière à ce que son extrémité de mesure soit reçue dans au moins un des logements d'insertion (31, 32, 33), cet élément de support modulaire (3) comportant des moyens de fixation de l'au moins un capteur (11, 12) aptes à maintenir l'au moins un capteur dans une position de mesure dans laquelle l'au moins un capteur (11, 12) est apte à mesurer l'au moins un paramètre physique, chimique ou biologique du fluide (9) s'écoulant à travers le conduit d'écoulement (22), chaque capteur (11, 12) étant connectés au module intelligent (4) par branchement sur un gestionnaire d'entrée-sortie (41).

2. Dispositif selon la revendication 1, **caractérisé en ce que** le module intelligent (4) comprend :
- des moyens de communication (5) aptes à réaliser une communication informatique entre le module intelligent et un terminal informatique distant, le module intelligent (4) étant agencé pour transmettre au terminal informatique une information relative à l'anomalie diagnostiquée.

3. Dispositif selon la revendication 2, **caractérisé en ce que** le module intelligent (4) comprend un composant apte à paramétrer ou configurer le dispositif par le terminal informatique distant à l'aide des moyens de communication (5).

4. Dispositif selon la revendication 3, **caractérisé en ce que** ledit composant est apte à paramétrer :
- une fréquence d'échantillonnage de l'au moins un capteur (11, 12), et/ou
- une fréquence de transmission de données de mesure vers le terminal informatique distant, et/ou
- un type de traitement ou une action lorsqu'un événement ou une anomalie est détectée.

5. Dispositif selon l'une quelconque des revendications précédentes, **caractérisé en ce que** l'au moins un capteur consiste en des capteurs.

6. Dispositif selon l'une des revendications précédentes, **caractérisé en ce qu'**il comprend une pile électrique (62) reliée à un module de gestion d'énergie (61) capable de transférer de l'énergie électrique au module intelligent (4).

7. Dispositif selon la revendication 6, **caractérisé en ce que** le module de gestion d'énergie (61) est relié à une batterie d'accumulateurs (62) et à un système de fourniture d'énergie additionnelle.

8. Dispositif selon la revendication 6, **caractérisé en ce que** le système de fourniture d'énergie additionnelle est agencé pour récupérer de l'énergie cinétique produite par l'écoulement du fluide (9) dans le conduit d'écoulement (22) à l'aide d'une turbine.

9. Dispositif selon la revendication 6, **caractérisé en ce que** le système de fourniture d'énergie additionnelle est agencé pour récupérer de l'énergie via un différentiel de température entre la conduite et un tampon d'un regard d'une chaussée.

10. Dispositif selon l'une des revendications précédentes, **caractérisé en ce que** la section interne moyenne (S) du conduit d'écoulement (22) est sensiblement identique à la section interne moyenne de la conduite.

11. Dispositif selon l'une des revendications 3 à 10, **caractérisé en ce que** le composant est apte à transmettre l'information relative à l'anomalie diagnostiquée dès que ladite anomalie est diagnostiquée.

12. Dispositif selon l'une des revendications 2 à 5, **caractérisé en ce que** les moyens de communication (5) comprennent au moins un émetteur compatible avec des systèmes de réception disponibles dans le périmètre géographique du réseau de distribution, autorisant une communication bidirectionnelle entre le module intelligent et le terminal informatique distant.

13. Dispositif selon l'une des revendications précédentes, **caractérisé en ce qu'**il comprend des moyens de paramétrage de la fréquence de mesure de l'au moins un capteur (11, 12), lesdits moyens de paramétrage pouvant paramétrer séparément cette fréquence de mesure pour chacun des capteurs (11, 12) lorsque le dispositif comprend plusieurs capteurs.

14. Dispositif selon l'une des revendications précédentes, **caractérisé en ce qu'**il comprend en outre un système de fourniture d'énergie additionnelle apte à récupérer de l'énergie présente dans l'environnement dans lequel est installé le dispositif, un système de conversion de cette énergie récupérée en énergie électrique et un système d'alimentation (61, 62) apte à alimenter le dispositif avec cette énergie électrique.

## Patentansprüche

1. Messvorrichtung für mindestens einen physikalischen, chemischen oder biologischen Parameter eines Fluids (9), insbesondere von Wasser oder Gas, das in einer zu einem Verteilungsnetz des Fluids gehörenden Leitung fließt, wobei diese Vorrichtung dazu bestimmt ist an dieser Leitung am Versorgungspunkt des Fluids installiert zu werden, sodass diese Leitung einen ersten und einen zweiten Abschnitt umfasst, wobei diese Vorrichtung umfasst:
- mindestens einen Sensor (11,12), der mit einer Messspitze versehen ist,
- einen Körper (21) mit einer Öffnung, die eine Abflussleitung (22) bildet, und mit mindestens einer Einsetzaufnahme (31,32,33) zur Aufnahme der Messspitze des mindestens einen Sensors (11,12), wobei die Einsetzaufnahme (31,32,33) in die Abflussleitung (22) mündet,
- Befestigungsmittel (221,222), die für folgende Anschlüsse angeordnet sind:
o eines ersten Endes (223) der Abflussleitung (22) mit einem Ende des ersten Abschnitts,
o eines zweiten Endes (224) der Abflussleitung (22) mit einem Ende des zweiten Abschnitts,
sodass das Fluid (9) durch die Abflussleitung (22) abfließen kann, und
ein intelligentes Modul (4), das mindestens eine Messung durch den mindestens einen Sensor zu bestimmten Zeitintervallen auslösen und eine Störung von mindestens einem physikalischen, chemischen oder biologischen Parameter des Fluids in Abhängigkeit mindestens eines Messwerts diagnostizieren kann,
**dadurch gekennzeichnet, dass** sie zudem ein modulares Halteelement (3) umfasst, das Öffnungen aufweist, die mit in etwa gleich großen Öffnungen in der Abflussleitung (22) fluchtend ausgerichtet sind, wobei diese Öffnungen Einsetzaufnahmen (31,32,33) sind, die angeordnet sind, um den mindestens einen Sensor (11,12) derart positionieren zu können, dass seine Messspitze in mindestens eine der Einsetzaufnahmen (31,32,33) aufgenommen werden kann, wobei dieses modulare Halteelement (3) Befestigungsmittel für den mindestens einen Sensor (11,12) aufweist, die den mindestens einen Sensor in einer Messposition halten können, in der der mindestens eine Sensor (11,12) den mindestens einen physikalischen, chemischen oder biologischen Parameter des Fluids (9) messen kann, das durch die Abflussleitung (22) fließt, wobei jeder Sensor (11,12) durch Anschluss an eine Ein-/Auslauf-Steuerung (41) mit dem intelligenten Modul (4) verbunden ist.

2. Vorrichtung nach Anspruch 1, **dadurch gekennzeichnet, dass** das intelligente Modul (4) umfasst:
- Kommunikationsmittel (5), die zwischen dem intelligenten Modul und einem entfernten IT-Endgerät eine IT-Kommunikation führen können, wobei das intelligente Modul (4) angeordnet ist, um dem IT-Endgerät eine Information bezüglich der diagnostizierten Störung zu übermitteln.

3. Vorrichtung nach Anspruch 2, **dadurch gekennzeichnet, dass** das intelligente Modul (4) ein Bauteil umfasst, das die Vorrichtung über das entfernte IT-Endgerät durch Kommunikationsmittel (5) parametrieren oder konfigurieren kann.

4. Vorrichtung nach Anspruch 3, **dadurch gekennzeichnet, dass** dieses Bauteil geeignet ist folgendes zu parametrieren:
- eine Frequenz der Probenahme des mindestens einen Sensors (11,12), und/oder
- eine Frequenz der Messdatenübertragung an das entfernte IT-Endgerät, und/oder
- eine Bearbeitungsart oder eine Aktion, wenn ein Ereignis oder eine Störung festgestellt wird.

5. Vorrichtung nach einem der vorhergehenden Ansprüche, **dadurch gekennzeichnet, dass** der mindestens eine Sensor aus Sensoren besteht.

6. Vorrichtung nach einem der vorhergehenden Ansprüche, **dadurch gekennzeichnet, dass** sie eine mit einem Energiesteuerungsmodul (61) verbundene elektrische Batterie (62) umfasst, die an das intelligente Modul (4) elektrische Energie übertragen kann.

7. Vorrichtung nach Anspruch 6, **dadurch gekennzeichnet, dass** das Energiesteuerungsmodul (61) mit einer Speicherbatterie (62) und einem zusätzlichen Energieversorgungssystem verbunden ist.

8. Vorrichtung nach Anspruch 6, **dadurch gekennzeichnet, dass** das zusätzliche Energieversorgungssystem angeordnet ist, um durch das Abfließen des Fluids (9) in der Abflussleitung (22) entstandene Bewegungsenergie mittels einer Turbine wiederzugewinnen.

9. Vorrichtung nach Anspruch 6, **dadurch gekennzeichnet, dass** das zusätzliche Energieversorgungssystem angeordnet ist, um aus einem Temperaturunterschied zwischen der Leitung und einem Puffer eines Straßenschachts entstandene Energie wiederzugewinnen.

10. Vorrichtung nach einem der vorhergehenden Ansprüche, **dadurch gekennzeichnet, dass** der durchschnittliche Innenquerschnitt (S) der Abflussleitung (22) in etwa dem durchschnittlichen Innenquerschnitt der Leitung entspricht.

11. Vorrichtung nach einem der Ansprüche 3 bis 10, **dadurch gekennzeichnet, dass** das Bauteil dazu fähig ist, die Information bezüglich der diagnostizierten Störung sofort nach deren Diagnose zu übermitteln.

12. Vorrichtung nach einem der Ansprüche 2 bis 5, **dadurch gekennzeichnet, dass** die Kommunikationsmittel (5) mindestens einen mit im geografischen Umfeld des Versorgungsnetzes verfügbaren Empfangssystemen kompatiblen Sender umfassen, sodass zwischen dem intelligenten Modul und dem entfernten IT-Endgerät eine bidirektionale Kommunikation möglich ist.

13. Vorrichtung nach einem der vorhergehenden Ansprüche, **dadurch gekennzeichnet, dass** sie Mittel zum Parametrieren der Messfrequenz des mindestens einen Sensors (11,12) umfasst, wobei die Mittel zum Parametrieren diese Messfrequenz für jeden der Sensoren (11,12) einzeln parametrieren können, wenn die Vorrichtung mehrere Sensoren umfasst.

14. Vorrichtung nach einem der vorhergehenden Ansprüche, **dadurch gekennzeichnet, dass** sie zudem ein zusätzliches Energieversorgungssystem umfasst, das dazu fähig ist, die in der Umgebung, in der die Vorrichtung installiert ist, vorhandene Energie rückzugewinnen, sowie ein Umwandlungssystem dieser rückgewonnenen Energie in elektrische Energie und ein Versorgungssystem (61,62), das dazu fähig ist, die Vorrichtung mit dieser elektrischen Energie zu versorgen.

## Claims

1. Device for measuring at least one physical, chemical or biological parameter of a fluid (9) in particular water or gas, flowing in a pipe belonging to a fluid distribution network, said device being intended to be installed as cutoff on said pipe at the point of delivery of the fluid, said pipe comprising a first section and a second section, said device comprising:
- at least one sensor (11, 12) provided with a measurement end;
- a body (21) comprising an opening forming a flow pipe (22) and at least one insertion housing (31, 32, 33) configured to receive said measurement end of the at least one sensor (11, 12), the insertion housing (31, 32, 33) emerging in the flow pipe (22);
- fixing means (221, 222) configured to connect:
o a first end (223) of the flow pipe (22) to an end of the first section,
o a second end (224) of the flow pipe (22) to an end of the second section,
so as to allow the fluid (9) to flow through the flow pipe (22) ; and
- a smart module (4) configured to:
trigger at least one measurement by the at least one sensor (11, 12) at determined time intervals, and
diagnose an anomaly of at least one physical, chemical or biological parameter of the fluid as a function of at least one measured value,
**characterized in that** it comprises further a modular support element (3) comprising orifices aligned with orifices of substantially the same dimensions realized in the flow pipe (22), these orifices which are insertion housings (31, 32, 33) being arranged to make it possible to position the at least one sensor (11, 12) in such a way that its measurement end is received in at least one of the insertion housings (31, 32, 33), this modular support element (3) comprising fixing means of the at least one sensor (11, 12) capable of holding the at least one sensor in a measurement position in which the at least one sensor (11, 12)is capable of measuring the at least one physical, chemical or biological parameter of the fluid (9) flowing through the flow pipe (22), each sensor (11, 12) being connected to the smart module (4) by connection to an input/output manager (41).

2. Device according to the claim 1, wherein said smart module (4) comprises :
- communication means (5) capable of carrying out a computerized communication between the smart module and a remote computer terminal, the smart module (4) being arranged to transmit to the computer terminal an information relating to the diagnosed anomaly.

3. Device according to the claim 2, wherein the smart module (4) comprises a component capable of parameterizing or configuring the device by the remote computer terminal using communication means (5).

4. Device according to the claim 3, wherein the said component is configured to parameterize :
- a sampling frequency of the at least one sensor (11, 12), and/or
- a frequency of transmission of measurement data to the remote computer terminal, and/or
- a type of processing or an action when an event or an anomaly is detected.

5. Device according any one of the preceding claims, wherein the at least one sensor consists of sensors.

6. Device according one of the preceding claims, further comprising an electric battery (62) linked to an energy management module (61) capable to transfer electrical energy to the smart module (4).

7. Device according to the claim 6, wherein the energy management module (61) is linked to a storage battery (62) and to an additional energy supply system.

8. Device according to the claim 6, wherein the additional energy supply system is configured to harvest kinetic energy produced by the flow of the fluid (9) in the flow pipe (22) using a turbine.

9. Device according to the claim 6, wherein the additional energy supply system is configured to harvest energy via a temperature differential between the pipe and a roadway manhole cover.

10. Device according one of the preceding claims, wherein the average internal section (S) of the flow pipe (22) is substantially identical to the average internal section of the pipe.

11. Device according one of the claims 3 to 10, wherein the component is capable of transmitting the information relating to the diagnosed anomaly by the instant at which said anomaly is diagnosed.

12. Device according one of the claims 2 to 5, wherein the communication means (5) comprise at least one transmitter compatible with receiver systems available within the geographic perimeter of the distribution network, allowing a two-way communication between the smart module and the remote computer terminal.

13. Device according one of the preceding claims, wherein it comprises parameterizing means of the measurement frequency of the at least one sensor (11, 12), said parameterizing means which can parameterize separately this measurement frequency for each of the sensors (11, 12) when the device comprises several sensors.

14. Device according one of the preceding claims, **characterized in that** it further comprises an additional energy supply system capable of harvesting energy present in the environment in which the device is installed, a system for converting this harvested energy into electrical energy and a power supply system (61, 62) capable of powering the device with this electrical energy.
